# EUROPEAN PATENT SPECIFICATION

(11) **EP 2 534 932 B1**
(45) Date of publication and mention of the grant of the patent: **20.08.2014**
(21) Application number: 11711634.3
(22) Date of filing: 09.02.2011
(51) Int. Cl.: H05K 3/10, G06K 19/077, H01F 41/06, H05K 13/06

(54) **APPARATUS FOR MANUFACTURING ANTENNAS FOR RADIO-FREQUENCY IDENTIFYING DEVICES**
VORRICHTUNG ZUR HERSTELLUNG VON ANTENNEN FÜR RFID-VORRICHTUNGEN
APPAREIL POUR LA FABRICATION D'ANTENNES POUR DISPOSITIFS D'IDENTIFICATION DE RADIOFRÉQUENCES

(30) Priority: 10.02.2010 IT MO20100025
(43) Date of publication of application: 19.12.2012
(73) Proprietor: SMART RES SOCIETA' PER AZIONI, 41057 Spilamberto (MO) (IT)
(72) Inventor: LOLLI, Marcello, i-41051 Castelnuovo Rangone (MO) (IT)
(74) Representative: Crugnola, Pietro
(86) International application number: PCT/IB2011/000230
(87) International publication number: WO 2011/098892

(56) References cited:
- WO-A1-2006/079913
- US-A- 4 450 623
- US-A1- 2001 011 413
- US-B1- 6 233 818

## Description

The present invention relates to an apparatus for manufacturing RIFD transponders, i.e. transponders for radio-frequency identifying devices, for example RIFD transponders operating at frequencies that are greater than 800 MHz (UHF). The RFID transponders consist of two essential parts, the socalled antenna, made of conductive material and an integrated circuit connected to the two ends of the antenna.

Between the conductive materials with which the antenna can be constructed it can be used copper wire.

Apparatuses are known for making copper wire antennas that lay and fix the wire on a substrate that is then used in subsequent machinings. Nevertheless, these apparatuses have a high cost/productivity ratio.

In fact, they consist of a certain number of heads, normally six, each of which, supplied by a spool of glazed copper wire, deposits the wire on a substrate, normally made of PVC. These heads move, tracing with the wire the drawing of the antenna and attaching the wire to the substrate by an ultrasonic frequency vibrating hammer that follows the head in its movement. The substrate has to be made either of a material that with the heat generated by the vibrating hammer is softened, retaining the wire, like the PVC, or has to be spread beforehand with a product that has the same function. The wire deposition heads have to be able to rotate by 360° because in the extension of the layout of the antenna the hammer always has to follow the laying of the wire. The heads that are thus constructed are relatively complex and heavy mechanisms, which entail that, both for economic questions and for questions of motion inertia, the number of heads that are applicable to an apparatus is relatively small. This causes a very high cost/productivity ratio.

From WO2006/079913 an apparatus according to the preamble of claim 1 is known for manufacturing an antenna for transponders made of wire made of electrically conductive material, which comprises a plurality of deposition heads for depositing said wire on a substrate and a supporting plane for said substrate that is movable in directions that are parallel to the axes of a Cartesian reference system.

Owing to the adoption of a supporting plane for the substrate that is movable according to the axes of a Cartesian reference system, it is possible to maintain the heads fixed with respect to said three axes. This enables the value of the moving masses to be maintained substantially constant, regardless of the number and the mass of the deposition means used, which enables productivity to be increased compared with apparatuses that adopt movable deposition heads. Nevertheless, also in this latter apparatus, there are significant moving masses, inasmuch as the entire supporting plane is moved along three axes of a Cartesian reference system, which limits the maximum speed at which the supporting plane can be moved, and, consequently, the productivity of the apparatus. Further relevant prior art is described in US 4450623, US 6233818 and US 2001/011413.

Object of the present invention is to provide an apparatus for manufacturing antennas for radio-frequency identifying devices in which moving masses.are minimised in order to maximise the production speed of said antennas. The object of the present invention is achieved with an apparatus according to claim 1.

Owing to the invention, the supporting plane of the substrate on which the antennas are made can be maintained fixed during manufacture of the antennas, which enables the speed of deposition of the wire constituting the antennas and thus the productivity of the apparatus according to the invention to be increased significantly compared with known prior art apparatuses. Further, movement of the movable parts of the apparatus, which are limited and of reduced weight, is obtained in an extremely simple and cheap manner.

One embodiment of the invention is illustrated by way of nonlimiting example in the following description, with reference to the attached drawings, in which:
Fig. 1 is a schematic plan view of an apparatus according to the invention;
Fig. 2 is a raised schematic view of the apparatus in Fig. 1;
Fig. 3 is a schematic view of a detail of the apparatus in
Fig. 1;

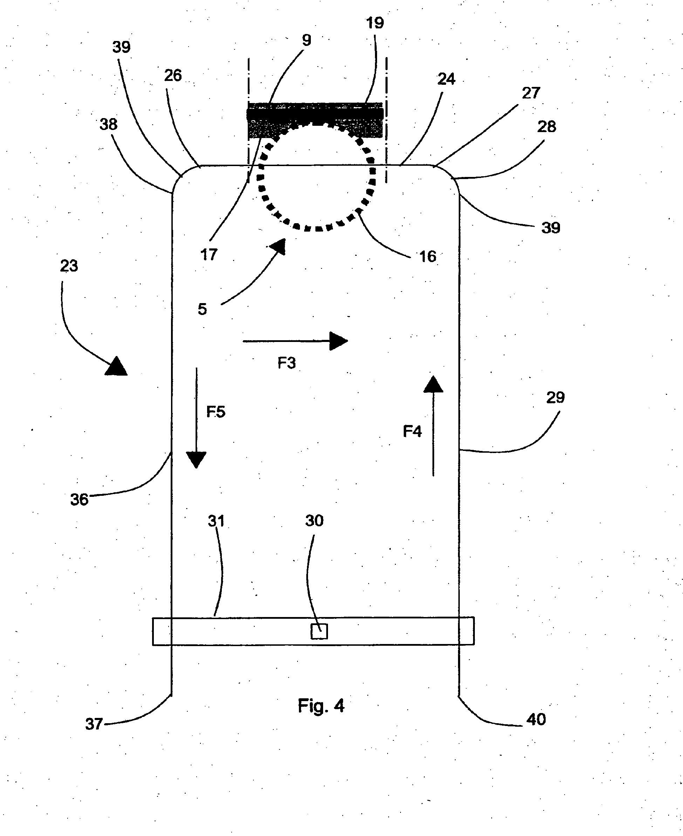

Fig. 4 schematically illustrates the procedure for making an
antenna with the apparatus according to the invention;

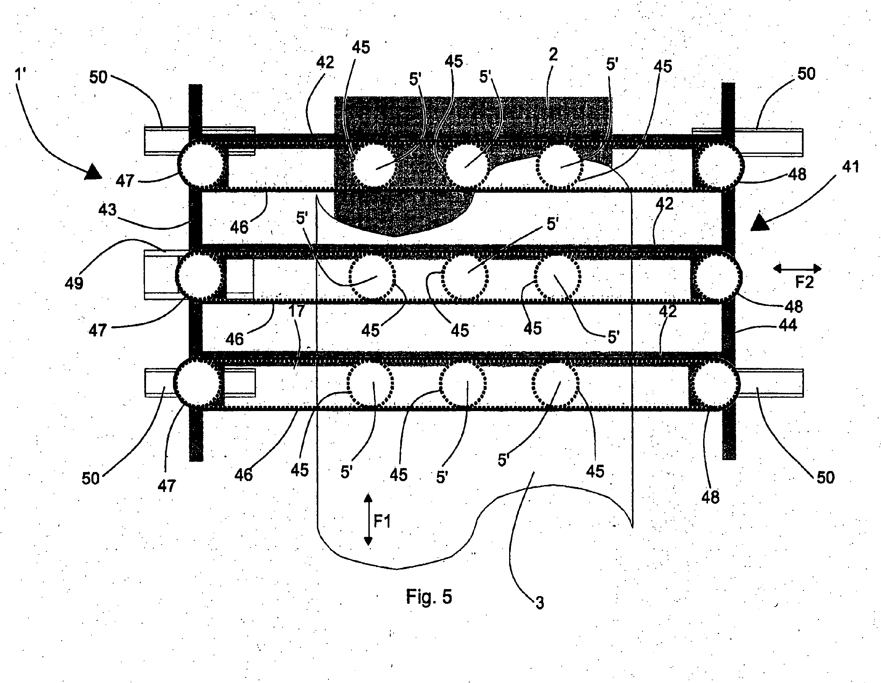

Fig. 5 is a schematic plan view of a version of the apparatus not forming part of the invention.

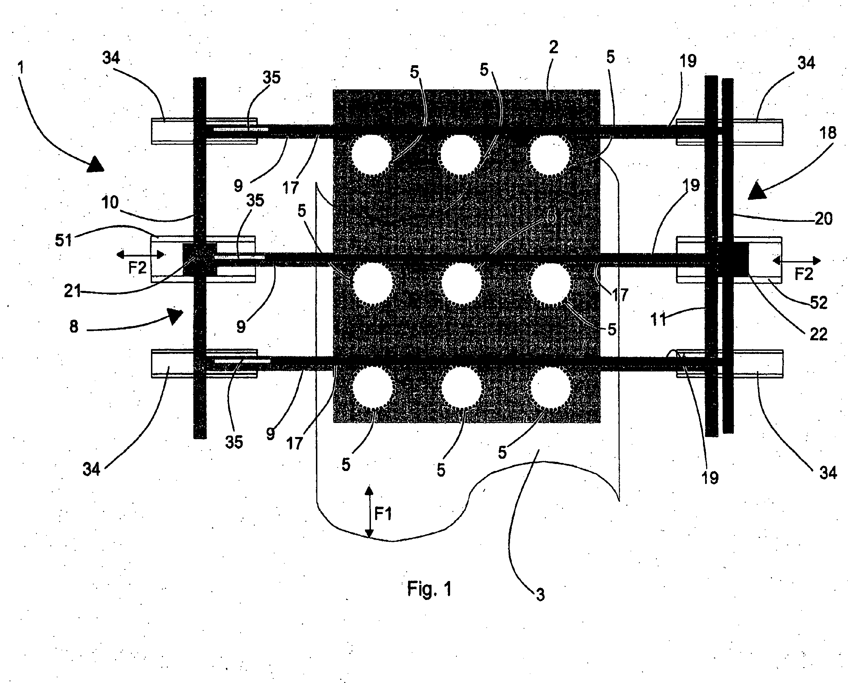

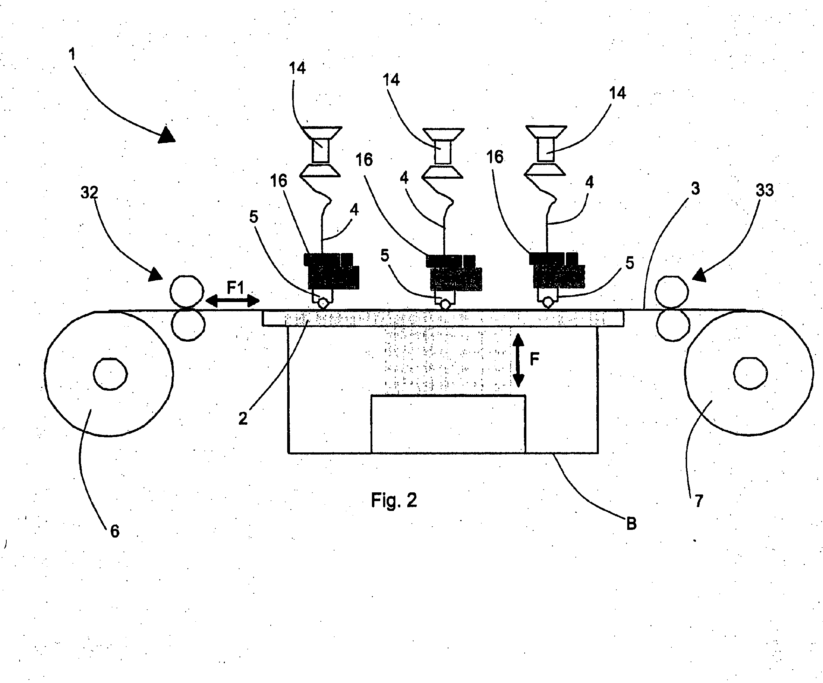

With reference to Figures 1 and 2, an apparatus 1 according to the invention comprises a base B with which a supporting plane 2 is associated that is vertically movable in a first direction F, on which a substrate 3 is slided in opposite directions along a second direction F1 by means of first dragging rollers 32 and second dragging rollers 33, a substrate 3, for example constituted by a web of paper or plastics, on which a plurality of antennas is made for radio-frequency identifying devices, in particular UHF frequency identifying devices. The second direction F1 is perpendicular to said first direction F.

The web 3 is unwound from a first reel 6 and wound on a second reel 7, after which said antennas are manufactured thereupon.

The antennas are manufactured by depositing and fixing on the substrate 3 a wire 4 made of electrically conductive material, for example a copper wire, which is laid on the substrate 3 in such a manner as to form the profile of the antenna.

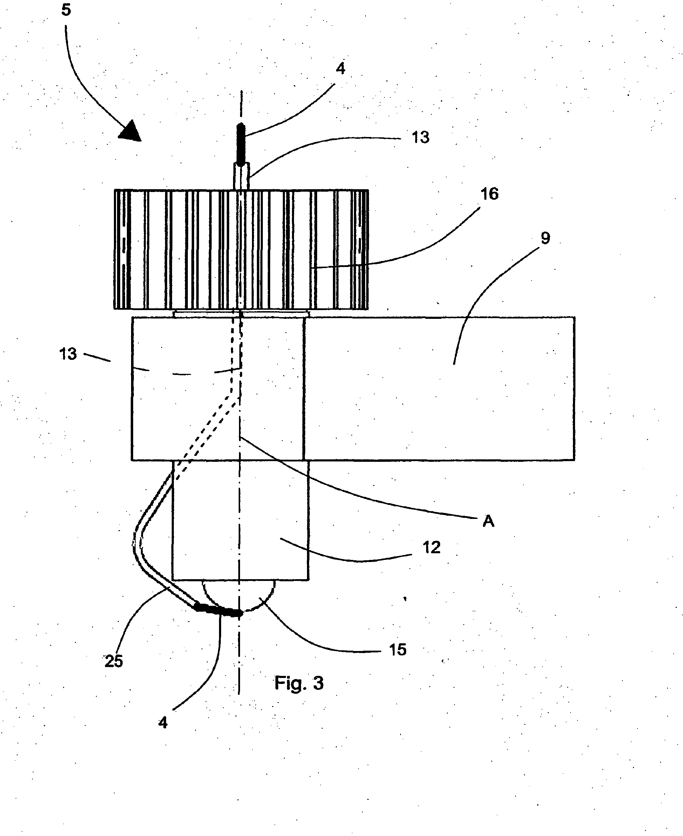

The wire 4 is deposited on the substrate 3 by a plurality of deposition devices 5. The deposition devices 5 are fixed to a first frame 8 consisting of a plurality of first arms 9 that are parallel to one another, arranged perpendicularly to said second direction F1 and connected at the respective ends to a respective first head 10 and to a respective second head 11. At least one deposition device 5, preferably a plurality of deposition devices 5, is fixed to each first arm 9. Each deposition device 5 is fixed to the respective first arm 9 such as to be able to rotate freely around a respective rotation axis A (Fig. 3) that is perpendicular to the supporting plane 2, i.e. parallel to said first direction F. The deposition device 5 (Fig. 3) comprises a guiding element 13 for guiding the wire 4, coming from a reel 14, through a body 12, and depositing the wire 4 on the substrate 3 through a dispensing end 25 of the guiding element 13, in the immediate vicinity of a pressing element 15 that presses on the substrate 3 the wire 4 in order to ensure the adhesion thereof to the substrate 3 to manufacture an antenna for an RFID device. On the substrate 3 a layer of adhesive substance is arranged, to obtain the adhesion of the wire 4 to the substrate 3.

A respective toothed wheel 16 is fixed to each body 12, the toothed wheel 16 being intended to engaged with a respective rack 17 that is fixed to a respective second arm 19 of a second frame 18. The set consisting of each body 12 with the respective toothed wheel 16 is supported on a respective first arm 9 of the first frame 8 such as to be able to rotate around the respective rotation axis A.

The second frame 18 comprises a plurality of second arms 19 that are parallel to one another and parallel to the first arms 9 of the first frame 8. One of the ends of the second arms 19 of the second frame 18 is connected to a respective third head 20.

The first frame 8 is driven to slide on first guides 34 that are fixed on the base B, by a first motor 21, for example a first linear motor or linear actuator, in a third direction F2 parallel to the supporting plane 2 and is perpendicular to said second direction F1.

The second frame 18 is driven to slide, in said third direction F2 parallel to the supporting plane 2, on second guides 35 that are obtained on the first arms 9 of the first frame 8, on which the corresponding second arms 19 of the second frame 18 slide. Driving the second frame 18 is obtained by a second motor 22, for example a second linear motor or linear actuator.

The first motor 21 and the second motor 22 can be fixed, respectively, to the first frame 8 and to the second frame 18 and may slide respectively on a third guide 51 and on a fourth guide 52 that are supported on the base B of the apparatus 1, to drive the first frame 8 and the second frame 18 to move along said direction F2.

In Figure 4 there is illustrated schematically the manufacture of an antenna 23 for a UHF frequency identifying device by the apparatus 1 according to the invention. A portion of substrate 3 is positioned on the supporting plane 2, which is then raised to bring the substrate 3 into contact with the dispensing ends 25 of the guiding elements 13.

The first frame 8 and the second frame 18 are positioned by the first motor 21 and the second motor 22 in such a manner that each deposition device 5 is located in an initial position 37 for depositing the wire 4 intended for constituting a first side 36 of the antenna 23, said initial position 37 corresponding to a first end of the first side 36.

Simultaneously, still via the first motor 21 and the second motor 22, the first frame 8 and the second frame 18 are moved in relation to one another, determining, through the effect of the engaging between the racks 17 and the toothed wheels 16, a rotation of the deposition devices 5 until orienting of the ends 25 of the guiding elements 13 is obtained that is suitable for dispensing the wire 4 along the direction of the first side 37 of the antenna 23.

After positioning of the first frame 8 and of the second frame 18 has been performed, the substrate 3 is slided along said second direction F1, in the direction of the arrow F5, whilst the first frame 8 and the second frame 18 are kept stationary, such that the wire 4 deposited by the deposition devices 5 makes on the substrate 3 a first side 36 of each antenna 23.

When each deposition device 5 reaches a final position 38, corresponding to a second end of the first side 36 of the antenna 23, the first frame 8 and the second frame 18 are moved in the direction of the arrow F3, at different speeds, such as to cause a rotation of the deposition devices 5 that enables the ends 25 of the guiding elements 13 to follow a trajectory corresponding to a first curved connection portion 39 between the first side 36 and a second side 24 of the antenna 23.

When the first curved connection portion 39 is completed, i.e. when the deposition devices 5 are in the position indicated by number 26 in Figure 4, corresponding to a first end of the second side 24 of the antenna 23, the motion of the substrate 3 is arrested whilst the first motor 21 and the second motor 22, are driven in such a manner as to move the first frame 8 and the second frame 18 at the same speed in the direction of the arrow F3 (Fig. 4), whilst the substrate 3 remains stationary, to deposit on the substrate 3 a portion of wire 4 intended for constituting the second side 24 of the antenna 23.

During this step of deposition of the wire 4, the dispensing devices 5 do not rotate around respective rotation axes A, inasmuch as the relative positions of the racks 17 and of the toothed wheels 16 remain fixed because there is no corresponding motion between the first frame 8 and the second frame 18.

When each deposition device 5 reaches the position indicated by the reference number 27 in figure 4, corresponding to a second end of the second side 24 of the antenna 23, the substrate 3 is moved in the direction of the arrow F4, whilst the first frame 8 and the second frame 18 continue to be moved in the direction of the arrow F3, but at different speeds, such as to cause a rotation of the deposition devices 5 that enables the ends 25 of the guiding elements 13 to follow a trajectory corresponding to a second curved connection portion 28 between the second side 24 and a third side 29 of the antenna 23.

When the second curved connection portion 28 is completed, the first frame 8 and the second frame 18 are arrested, whilst the substrate 3 continues to slide along said second direction F1, in the direction of the arrow F4, such that the wire deposited by the deposition devices 5 makes on the substrate 3 the third side 29 of each antenna 23. At the end of manufacture of the antennas 23, the wire 4 is cut by a respective cutting device and the apparatus 1 is ready for manufacturing a successive series of antennas 23 on the substrate 3.

The first side 36 and the third side 29 of each antenna 23 are deposited on the substrate 3 such as to be superimposed, near, respectively, the first end 37 and the second end 40 of the antenna 23, on a respective modular connecting element 31 that is preventively fixed in a preset position on the substrate 3 and to which a microchip 30 is connected. At the end of the deposition of the antennas 23, the respective end portions thereof are welded to the respective modular elements 31 to complete the radio-frequency identifying devices.

After completing of a group of radio-frequency identifying devices, the support 2 is lowered by moving the substrate 3 away from the deposition devices 5 and the substrate 3 is advanced for a preset distance in the direction F1 in the direction of the arrow F4 or F5, to position a new group of modular elements 31 in preset positions with respect to the deposition devices 5 to deposit a new group of antennas 23 on the substrate 3 and connect the new group of antennas 23 to the new group of modular elements 31. Lastly, the supporting element 2 is raised until the substrate 3 is brought into contact with the deposition devices 5 to start the deposition of a new group of antennas on the substrate 3. During deposition of the wire 4 to manufacture the antennas 23, the only moving masses consist of the first frame 8, the devices 5 for depositing the wire 4 and the second frame 18.

The moving masses are supported on the base B and have a much lower value than the moving masses in known prior art apparatuses. Further, during manufacture of the sides of the antenna 23 parallel to the second direction F1, the first frame 8 and the second frame 18 remain in a fixed position, i.e. they do not move, whilst everything that moves is only the substrate 3 on which is deposited the wire 4, which moves along said second direction F1, in the direction of the arrow F4, or in the direction of the arrow F5. This all enables a significant increase in the speed of deposition of the wire, and thus, of productivity, compared with known prior art apparatuses. Further, the energy consumption of the apparatus 1 is much lower, at the same level of productivity, than the energy consumption of the known prior art apparatuses.

In Figure 5 there is illustrated a version 1' of the apparatus 1 not forming part of the invention.

In this version 1', deposition devices 5' of the wire 4, which are similar to the deposition devices 5 illustrated in Figure 3, are fixed to a frame 41 consisting of a plurality of arms 42 that are parallel to one another, arranged perpendicularly to said second direction F1 and connected, at the respective ends, to a respective first head 43 and to a respective second head 44. The frame 41 is slidable on guides 50 that are fixed on the base B and is moved on said guides 50, in said direction F1 by a motor 49, for example a linear motor or linear actuator.

At least one deposition device 5', preferably a plurality of deposition devices 5' is fixed to each arm 42. Each deposition device 5' is fixed to the respective arm 42 such as to be able to rotate freely around a respective rotation axis A that is perpendicular to the supporting plane 2, i.e. parallel to said first direction F.

The deposition devices 5' differ form the deposition devices 5 through the fact that to each body 12, instead of a respective toothed wheel 16, a respective toothed pulley 45 is fixed that is intended for engaging with a respective toothed belt 46 that winds on a respective driving pulley 47 and on a respective driven pulley 48 that are respectively supported on the first head 43 and on the second head 44 of the frame 41. Each driving pulley 47 can be driven by a respective motor. Alternatively, all the driving pulleys 47 can be driven by a single motor via a further belt transmission (not shown). The set consisting of each body 12 with the respective toothed pulley 45 is supported on a respective arm 42 of the frame 41 so as to be able to rotate around the respective rotation axis A.

In the version of the apparatus 1 illustrated in Figure 5, the rotation of the deposition devices 5' necessary for making the curved connections between the sides of the antenna 23 is obtained via the toothed belts 46 whilst the movement of the deposition devices in the direction F1 is obtained by moving the frame 41 in said direction. For the rest, the operation of the version 1' is identical to the operation of the apparatus 1 disclosed with reference to Figures 1 to 4.

The rotation movement of the deposition devices 5' by the toothed belts 46 enables antennas of a complex form to be manufactured that are difficult to manufacture with the embodiment of the apparatus disclosed in Figures 1 to 4, for example antennas with a spiral form.

In the practical embodiment, the materials, dimensions and constructional details may be different from those indicated but be technically equivalent thereto without thereby falling outside the scope of the present invention.

## Claims

1. Apparatus (1) for manufacturing antennas (23) for radio-frequency identifying devices, comprising a supporting plane (2) vertically movable in a first direction (F), intended to support a substrate (3) on which said antennas (23) are manufactured, at least one deposition, device (5) for deposition of a wire (4) made of electrically conducting material intended to constitute said antennas (23), a first frame (8) which supports said at least one deposition device (5 ) so that said deposition device (5) is free to rotated around a respective rotation axis (A) perpendicular to said supporting plane (2), said first frame (8) being movable along a third direction (F2) parallel to said supporting plane (2), rotation driving means (16, 17) suitable to rotate said at least one deposition device (5) around said axis (A), **characterized in that** said apparatus (1) further comprises dragging means (32; 33) suitable for sliding said substrate (3) in opposite directions along a second direction (F1) parallel to said supporting plane (2) and perpendicular to said third direction (F2) and said first direction (F) and a second frame (18) associated with said first frame (8) and movable along said third direction (F2); said at least one first frame (8) comprising a plurality of first arms (9) to each of which is supported at least one said deposition device (5); said second frame (18) comprising a plurality of second arms (19) parallel to said first arms (9); said rotation driving means (16, 17) comprising rack means (17) associated with each of said second arms (19) and gear means (16) engaging with said rack means (17), each deposition device (5) being associated with one of said gear means (16).

2. Apparatus (1) according to claim 1, wherein said first arms (9) are fixed at the respective ends to a respective first head (10) and a respective second head (11).

3. Apparatus (1) according to any one of the preceding claims, wherein said at least one first frame (8) is drivable to move along said third direction (F2) by first actuator means (21).

4. Apparatus (1) according to any one of the preceding claims, wherein said at least one first frame is slidable on first guides (34, 35) fixed to a base (B) of the apparatus (1).

5. Apparatus (1) according to one of preceding claims, wherein said second frame (18) is drivable to move along said third direction (F2) by second actuator means (22).

6. Apparatus (1) according to one of preceding claims, wherein said second frame (18) is slidable on second guides (35) obtained on the first arms (9) of the first frame (8).

7. Apparatus (1) according to any one of the preceding claims, wherein each of said deposition devices (5) comprises a body (12), a guide element (13) suitable for guiding said wire (4) through said body (12) and dispensing said wire (4) from a dispensing end (25) and pressure means (15) suitable for pressing on said substrate (3) the wire (4) dispensed by said dispensing end (25).

8. Apparatus (1) according to claim 7, wherein said gear means (16) are fixed to said body (12).

9. Apparatus (1) according to claim 7 or 8, wherein said body (12) is supported at a respective first arm (9) so as to be rotatable around a respective rotation axis (A).

## Patentansprüche

1. Apparatur (1) zur Herstellung von Antennen (23) für Vorrichtungen zur Identifizierung von Radiofrequenzen, die Folgendes aufweist, nämlich eine Tragfläche (2), die vertikal in eine erste Richtung (F) bewegbar ist, die dazu vorgesehen ist, ein Substrat (3) zu tragen, auf dem die Antennen (23) hergestellt werden, zumindest eine Ablegevorrichtung (5) zum Ablegen eines Drahtes (4), der aus elektrisch leitfähigem Material hergestellt und dazu vorgesehen ist, die Antennen (23) zu bilden, einen ersten Rahmen (8), der die zumindest eine Ablegevorrichtung (5) trägt, so dass die Ablegevorrichtung (5) dazu frei ist, um eine jeweilige Rotationsachse (A) zu rotieren, die rechtwinklig ist zu der Tragfläche (2), wobei der erste Rahmen (8) entlang einer dritten Richtung (F2) bewegbar ist, die parallel zu der Tragfläche (2) ist, Rotationsantriebsmittel (16, 17), die geeignet sind, um die zumindest eine Ablegevorrichtung (5) um die Achse (A) zu rotieren, **dadurch gekennzeichnet, dass** die Apparatur (1) ferner Folgendes aufweist, nämlich Zugmittel (32, 33), die geeignet sind, um das Substrat (3) in entgegengesetzte Richtungen entlang einer zweiten Richtung (F1) gleiten zu lassen, die parallel zu der Tragfläche (2) und rechtwinklig zu der dritten Richtung (F2) und der ersten Richtung (F) ist, und einen zweiten Rahmen (18), der dem ersten Rahmen (8) zugeordnet ist und entlang der dritten Richtung (F2) bewegbar ist; wobei der zumindest eine erste Rahmen (8) eine Vielzahl von ersten Armen (9) aufweist, an denen jeweils zumindest eine der Ablegevorrichtungen (5) getragen wird; wobei der zweite Rahmen (18) eine Vielzahl von zweiten Armen (19) aufweist, die parallel sind zu den ersten Armen (9); wobei die Rotationsantriebsmittel (16, 17) Gestellmittel (17), die jedem der zweiten Arme (19) zugeordnet sind, und Eingriffsmittel (16) aufweisen, die mit den Gestellmitteln (17) in Eingriff stehen, wobei jede Ablegevorrichtung (5) einem der Eingriffsmittel (16) zugeordnet ist.

2. Apparatur (1) gemäß Anspruch 1, wobei die ersten Arme (9) an den jeweiligen Enden mit einem jeweiligen ersten Kopf (10) und einem jeweiligen zweiten Kopf (11) befestigt sind.

3. Apparatur (1) gemäß einem der vorherigen Ansprüche, wobei der zumindest eine erste Rahmen (8) antreibbar ist, um sich entlang der dritten Richtung (F2) durch erste Aktuatormittel (21) zu bewegen.

4. Apparatur (1) gemäß einem der vorherigen Ansprüche, wobei der zumindest erste Rahmen auf ersten Führungen (34, 35) gleitbar ist, die an einer Basis (B) der Apparatur (1) befestigt sind.

5. Apparatur (1) gemäß einem der vorherigen Ansprüche, wobei der zweite Rahmen (18) antreibbar ist, um sich entlang der dritten Richtung (F2) durch zweite Aktuatormittel (22) zu bewegen.

6. Apparatur (1) gemäß einem der vorherigen Ansprüche, wobei der zweite Rahmen (18) auf zweiten Führungen (35) gleitbar ist, die auf den ersten Armen (9) des ersten Rahmens (8) vorgesehen sind.

7. Apparatur (1) gemäß einem der vorherigen Ansprüche, wobei jede der Ablegevorrichtungen (5) einen Körper (2), ein Führungselement (13), das geeignet ist, um den Draht (4) durch den Körper (12) zu führen und den Draht (4) aus einem Ausgabeende (25) auszugeben, und Druckmittel (15) aufweist, die geeignet sind, um den Draht (4), der von dem Ausgabeende (25) ausgegeben wird, auf das Substrat (3) zu drücken.

8. Apparatur (1) gemäß Anspruch 7, wobei die Eingriffsmittel (16) an dem Körper (12) befestigt sind.

9. Apparatur (1) gemäß Anspruch 7 oder 8, wobei der Körper (12) an einem jeweiligen ersten Arm (9) getragen wird, so dass dieser um eine jeweilige Rotationsachse (A) rotierbar ist.

## Revendications

1. Appareil (1) pour la fabrication d'antennes (23) pour des dispositifs d'identification de radiofréquences, comprenant un plan de support (2) mobile verticalement dans une première direction (F) et destiné à porter un substrat (3) sur lequel lesdites antennes (23) sont fabriquées, au moins un dispositif de dépôt (5) pour le dépôt d'un fil (4) réalisé en un matériau électriquement conducteur et destiné à constituer lesdites antennes (23), un premier cadre (8) qui porte ledit au moins un dispositif de dépôt (5) de telle manière que ledit dispositif de dépôt (5) est libre de tourner autour d'un axe de rotation respectif (A) perpendiculaire audit plan de support (2), ledit premier cadre (8) étant mobile le long d'une troisième direction (F2) parallèle audit plan de support (2), des moyens d'entraînement en rotation (16, 17) aptes à faire tourner ledit au moins un dispositif de dépôt (5) autour dudit axe (A), **caractérisé en ce que** ledit appareil (1) comprend en outre des moyens de traînage (32 ; 33) aptes à faire coulisser ledit substrat (3) dans des directions opposées le long d'une deuxième direction (F1) parallèle audit plan de support (2) et perpendiculaire à ladite troisième direction (F2) et à ladite première direction (F), et un deuxième cadre (18) associé audit premier cadre (8) et mobile le long de ladite troisième direction (F2) ; ledit au moins un premier cadre (8) comprenant une pluralité de premiers bras (9), chacun de ces derniers portant ledit au moins un dispositif de dépôt (5) ; ledit deuxième cadre (18) comprenant une pluralité de deuxièmes bras (19) parallèles auxdits premiers bras (9) ; lesdits moyens d'entraînement en rotation (16, 17) comprenant des moyens de crémaillère (17) associés à chacun desdits deuxièmes bras (19) et à des moyens d'engrenage (16) venant en engagement avec lesdits moyens de crémaillère (17), chaque dispositif de dépôt (5) étant associé à l'un desdits moyens d'engrenage (16).

2. Appareil (1) selon la revendication 1, dans lequel lesdits premiers bras (9) sont fixés, aux extrémités respectives, à une première tête respective (10) et une deuxième tête respective (11).

3. Appareil (1) selon l'une quelconque des revendications précédentes, dans lequel ledit au moins un premier cadre (8) est apte à être entraîné, par des premiers moyens d'actionnement (21), à se déplacer le long de ladite troisième direction (F2).

4. Appareil (1) selon l'une quelconque des revendications précédentes, dans lequel ledit au moins un premier cadre est apte à coulisser sur des premiers guides (34, 35) fixés à une base (B) de l'appareil (1).

5. Appareil (1) selon une des revendications précédentes, dans lequel ledit deuxième cadre (18) est apte à être entraîné, par des deuxièmes moyens d'actionnement (22), à se déplacer le long de ladite troisième direction (F2).

6. Appareil (1) selon une des revendications précédentes, dans lequel ledit deuxième cadre (18) est apte à coulisser sur des deuxièmes guides (35) obtenus sur les premiers bras (9) du premier cadre (8).

7. Appareil (1) selon l'une quelconque des revendications précédentes, dans lequel chacun desdits dispositifs de dépôt (5) comprend un corps (12), un élément de guidage (13) apte à guider ledit fil (4) à travers ledit corps (12) et à distribuer ledit fil (4) à partir d'une extrémité de distribution (25), et des moyens de pression (15) aptes à comprimer sur ledit substrat (3) le fil (4) distribué par ladite extrémité de distribution (25).

8. Appareil (1) selon la revendication 7, dans lequel lesdits moyens d'engrenage (16) sont fixés audit corps (12).

9. Appareil (1) selon la revendication 7 ou 8, dans lequel ledit corps (12) est porté au niveau d'un premier bras respectif (9) de manière à pouvoir tourner autour d'un axe de rotation respectif (A).
